# EUROPÄISCHE PATENTSCHRIFT

(11) **EP 2 470 221 B1**
(45) Veröffentlichungstag und Bekanntmachung des Hinweises auf die Patenterteilung: **23.03.2016**
(21) Anmeldenummer: 10744904.3
(22) Anmeldetag: 12.08.2010
(51) Int. Cl.: A61L 15/18, A61L 15/46, A61L 15/60, A61F 13/84

(54) **GERUCHSINHIBIERENDE ZUSAMMENSETZUNGEN**
DEODORIZING COMPOSITIONS
COMPOSITIONS INHIBANT LES ODEURS

(30) Priorität: 26.08.2009 US 236896 P
(43) Veröffentlichungstag der Anmeldung: 04.07.2012
(73) Patentinhaber: BASF SE, 67056 Ludwigshafen (DE)
(72) Erfinder: BRAIG, Volker, 69469 Weinheim-Lützelsachsen (DE); DANIEL, Thomas, 67165 Waldsee (DE); KONRADI, Rupert, 68526 Ladenburg (DE); PLATSCH, Herbert, 68259 Mannheim (DE); SOBOTKA, Bettina, 68165 Mannheim (DE); JENTZSCH, Axel, 67063 Ludwigshafen (DE)
(86) Internationale Anmeldenummer: PCT/EP2010/061719
(87) Internationale Veröffentlichungsnummer: WO 2011/023560

(56) Entgegenhaltungen:
- EP-A1- 1 034 800
- WO-A1-03/095510
- WO-A1-2009/101060
- WO-A2-2007/104641
- WO-A2-2010/096595
- GB-A- 627 512
- US-A- 3 783 872
- US-A- 4 103 062
- US-A- 4 363 322
- US-A- 4 381 783

## Beschreibung

Die vorliegende Erfindung betrifft geruchsinhibierende Zusammensetzungen, enthaltend Superabsorberpartikel und Metallperoxide, sowie deren Herstellung.

Superabsorberpartikel werden zur Herstellung von Windeln, Tampons, Damenbinden und anderen Hygieneartikeln, aber auch als wasserzurückhaltende Mittel im landwirtschaftlichen Gartenbau verwendet.

Die Herstellung von Superabsorberpartikeln wird in der Monographie "Modern Superabsorbent Polymer Technology", F.L. Buchholz und A.T. Graham, Wiley-VCH, 1998, Seiten 71 bis 103, beschrieben. Ullmann's Encyclopedia of Industrial Chemistry, 6. Auflage 2003, ISBN 3-527-30385-5, Stichwort "Superabsorbents" fasst als Teil eines enzyklopädisches Übersichtswerks über die gesamte chemische Technik das ganz grundlegende Fachwissen auf diesem Gebiet zusammen.

Die Eigenschaften der Superabsorberpartikel können beispielsweise über die verwendete Vernetzermenge eingestellt werden. Mit steigender Vernetzermenge sinkt die Zentrifugenretentionskapazität (CRC) und die Absorption unter einem Druck von 21,0 g/cm² (AUL0.3psi) durchläuft ein Maximum.

Zur Verbesserung der Anwendungseigenschaften, wie beispielsweise Permeabilität des gequollenen Gelbetts (SFC) in der Windel und Absorption unter einem Druck von 49.2 g/cm² (AUL0.7psi), werden Superabsorberpartikel im allgemeinen oberflächennachvernetzt. Dadurch steigt der Vernetzungsgrad der Partikeloberfläche, wodurch die Absorption unter einem Druck von 49,2 g/cm² (AUL0.7psi) und die Zentrifugenretentionskapazität (CRC) zumindest teilweise entkoppelt werden können. Diese Oberflächennachvernetzung kann in wässriger Gelphase durchgeführt werden. Vorzugsweise werden aber getrocknete, gemahlene und abgesiebte Polymerpartikel (Grundpolymer) an der Oberfläche mit einem Oberflächennachvernetzer beschichtet, thermisch oberflächennachvernetzt und getrocknet. Dazu geeignete Vernetzer sind Verbindungen, die mit mindestens zwei Carboxylatgruppen der Superabsorberpartikel kovalente Bindungen bilden können.

JP 2001/39802 lehrt die Verwendung von Natriumperkarbonat und Natriumperborat als antimikrobielle Zusätze für wasserabsorbierende Zusammensetzungen.

GB 627,512 offenbart die Verwendung von Zinkperoxid zur Geruchsinhibierung in Hygieneartikeln.

GB 2 377 890 beschreibt Oxidationsmittel als geruchsinhibierende Zusatzstoffe in wasserabsorbierenden Zusammensetzungen.

JP 2001/115042 offenbart wasserabsorbierende Zusammensetzungen, enthaltend Superabsorberpartikel, anorganische Peroxide und Ethylendiamintetraessigsäure.

US 4 363 322 beschreibt Hygieneartikel oder Wundauflagen, die neben absorbierendem Pulver auch Peroxid enthalten.

WO 2009/10 160 A1 lehrt Zusammensetzungen aus Superabsorberpartikeln und geruchsinhibieren-den pflanzlichen Stoffen.

WO 2010/096 595 A1 offenbart Superabsorberpartikel, denen Wasserstoffperoxid und optional anschließend oder auch in einer Lösung Metallsalz zugegeben wird.

Aufgabe der vorliegenden Erfindung war die Bereitstellung verbesserter geruchsinhibierender Zusammensetzungen, insbesondere von geruchsinhibierenden Zusammensetzungen mit verbesserter Lagerstabilität.

Gelöst wurde die Aufgabe durch geruchsinhibierende Zusammensetzungen, enthaltend Superabsorberpartikel und mindestens ein Metallperoxid, das Zinkperoxid, Magnesiumperoxid oder Kalziumperoxid ist. Bevorzugt ist Zinkperoxid.

Die erfindungsgemäße Zusammensetzung enthält vorzugsweise 0,001 bis 5 Gew.-%, bevorzugt von 0,01 bis 3 Gew.-%, besonders bevorzugt von 0,1 bis 1,5 Gew.-%, ganz besonders bevorzugt von 0,2 bis 0,8 Gew.-%, des Metallperoxids.

Die erfindungsgemäße Zusammensetzung enthält vorzugsweise weniger als 10 Gew.-%, bevorzugt weniger als 8 Gew.-%, besonders bevorzugt weniger als 6 Gew.-%, ganz besonders bevorzugt weniger als 5 Gew.-%, Wasser.

Die erfindungsgemäße Zusammensetzung enthält vorzugsweise mindestens 90 Gew.-%, besonders bevorzugt mindestens 95 Gew.-%, bevorzugt mindestens 97 Gew.-%, ganz besonders bevorzugt mindestens 98 Gew.-%, Superabsorberpartikel.

Der vorliegenden Erfindung liegt die Erkenntnis zugrunde, dass Metallperoxide, insbesondere Zinkperoxid, eine gute geruchsinhibierende Wirkung und die damit hergestellten Zusammensetzungen eine hohe Lagerstabilität aufweisen.

Die erfindungsgemäßen Zusammensetzungen enthalten vorzugsweise weniger als 1 ppm, besonders bevorzugt weniger als 10 ppm, ganz besonders bevorzugt weniger als 5 ppm, Schwermetallionen. Schwermetallionen, insbesondere Eisenionen, führen zur katalytischen Zersetzung der Metallperoxide und senken somit die Lagerstabilität der erfindungsgemäßen Zusammensetzungen.

Im Folgenden wird die Herstellung der Superabsorberpartikel näher erläutert.

Die Superabsorberpartikel werden beispielsweise durch Polymerisation einer Monomerlösung oder -suspension, enthaltend
a) mindestens ein ethylenisch ungesättigtes, säuregruppentragendes Monomer, das zumindest teilweise neutralisiert sein kann,
b) mindestens einen Vernetzer,
c) mindestens einen Initiator,
d) optional ein oder mehrere mit den unter a) genannten Monomeren copolymerisierbare ethylenisch ungesättigte Monomere und
e) optional ein oder mehrere wasserlösliche Polymere,
hergestellt und sind üblicherweise wasserunlöslich.

Die Monomeren a) sind vorzugsweise wasserlöslich, d.h. die Löslichkeit in Wasser bei 23°C beträgt typischerweise mindestens 1 g/100 g Wasser, vorzugsweise mindestens 5 g/100 g Wasser, besonders bevorzugt mindestens 25 g/100 g Wasser, ganz besonders bevorzugt mindestens 35 g/100 g Wasser.

Geeignete Monomere a) sind beispielsweise ethylenisch ungesättigte Carbonsäuren, wie Acrylsäure, Methacrylsäure, und Itaconsäure. Besonders bevorzugte Monomere sind Acrylsäure und Methacrylsäure. Ganz besonders bevorzugt ist Acrylsäure.

Weitere geeignete Monomere a) sind beispielsweise ethylenisch ungesättigte Sulfonsäuren, wie Styrolsulfonsäure und 2-Acrylamido-2-methylpropansulfonsäure (AMPS).

Verunreinigungen können einen erheblichen Einfluss auf die Polymerisation haben. Daher sollten die eingesetzten Rohstoffe eine möglichst hohe Reinheit aufweisen. Es ist daher oft vorteilhaft die Monomeren a) speziell zu reinigen. Geeignete Reinigungsverfahren werden beispielsweise in der WO 2002/055469 A1, der WO 2003/078378 A1 und der WO 2004/035514 A1 beschrieben. Ein geeignetes Monomer a) ist beispielsweise eine gemäß WO 2004/035514 A1 gereinigte Acrylsäure mit 99,8460 Gew.-% Acrylsäure, 0,0950 Gew.-% Essigsäure, 0,0332 Gew.-% Wasser, 0,0203 Gew.-% Propionsäure, 0,0001 Gew.-% Furfurale, 0,0001 Gew.-% Maleinsäureanhydrid, 0,0003 Gew.-% Diacrylsäure und 0,0050 Gew.-% Hydrochinonmonomethylether. Der Anteil an Acrylsäure und/oder deren Salzen an der Gesamtmenge der Monomeren a) beträgt vorzugsweise mindestens 50 mol-%, besonders bevorzugt mindestens 90 mol-%, ganz besonders bevorzugt mindestens 95 mol-%.

Die Monomere a) enthalten üblicherweise Polymerisationsinhibitoren, vorzugsweise Hydrochinonhalbether, als Lagerstabilisator.

Die Monomerlösung enthält vorzugsweise bis zu 250 Gew.-ppm, bevorzugt höchstens 130 Gew.-ppm, besonders bevorzugt höchstens 70 Gew.-ppm, bevorzugt mindestens 10 Gew.-ppm, besonders bevorzugt mindestens 30 Gew.-ppm, insbesondere um 50 Gew.-ppm, Hydrochinonhalbether, jeweils bezogen auf das unneutralisierte Monomer a). Beispielsweise kann zur Herstellung der Monomerlösung ein ethylenisch ungesättigtes, säuregruppentragendes Monomer mit einem entsprechenden Gehalt an Hydrochinonhalbether verwendet werden.

Bevorzugte Hydrochinonhalbether sind Hydrochinonmonomethylether (MEHQ) und/oder alpha-Tocopherol (Vitamin E).

Geeignete Vernetzer b) sind Verbindungen mit mindestens zwei zur Vernetzung geeigneten Gruppen. Derartige Gruppen sind beispielsweise ethylenisch ungesättigte Gruppen, die in die Polymerkette radikalisch einpolymerisiert werden können, und funktionelle Gruppen, die mit den Säuregruppen des Monomeren a) kovalente Bindungen ausbilden können. Weiterhin sind auch polyvalente Metallsalze, die mit mindestens zwei Säuregruppen des Monomeren a) koordinative Bindungen ausbilden können, als Vernetzer b) geeignet.

Vernetzer b) sind vorzugsweise Verbindungen mit mindestens zwei polymerisierbaren Gruppen, die in das Polymernetzwerk radikalisch einpolymerisiert werden können. Geeignete Vernetzer b) sind beispielsweise Ethylenglykoldimethacrylat, Diethylenglykoldiacrylat, Polyethylenglykoldiacrylat, Allylmethacrylat, Trimethylolpropantriacrylat, Triallylamin, Tetraallylammoniumchlorid, Tetraallyloxyethan, wie in EP 0 530 438 A1 beschrieben, Di- und Triacrylate, wie in EP 0 547 847 A1, EP 0 559 476 A1, EP 0 632 068 A1, WO 93/21237 A1, WO 2003/104299 A1, WO 2003/104300 A1, WO 2003/104301 A1 und DE 103 31 450 A1 beschrieben, gemischte Acrylate, die neben Acrylatgruppen weitere ethylenisch ungesättigte Gruppen enthalten, wie in DE 103 31 456 A1 und DE 103 55 401 A1 beschrieben, oder Vernetzermischungen, wie beispielsweise in DE 195 43 368 A1, DE 196 46 484 A1, WO 90/15830 A1 und WO 2002/032962 A2 beschrieben.

Bevorzugte Vernetzer b) sind Pentaerythrittriallylether, Tetraalloxyethan, Methylenbismethacrylamid, 15-fach ethoxiliertes Trimethylolpropantriacrylat, Polyethylenglykoldiacrylat, Trimethylolpropantriacrylat und Triallylamin.

Ganz besonders bevorzugte Vernetzer b) sind die mit Acrylsäure oder Methacrylsäure zu Di- oder Triacrylaten veresterten mehrfach ethoxylierten und/oder propoxylierten Glyzerine, wie sie beispielsweise in WO 2003/104301 A1 beschrieben sind. Besonders vorteilhaft sind Di- und/oder Triacrylate des 3- bis 10-fach ethoxylierten Glyzerins. Ganz besonders bevorzugt sind Di- oder Triacrylate des 1- bis 5-fach ethoxylierten und/oder propoxylierten Glyzerins. Am meisten bevorzugt sind die Triacrylate des 3- bis 5-fach ethoxylierten und/oder propoxylierten Glyzerins, insbesondere das Triacrylat des 3-fach ethoxylierten Glyzerins.

Die Menge an Vernetzer b) beträgt vorzugsweise 0,05 bis 1,5 Gew.-%, besonders bevorzugt 0,1 bis 1 Gew.-%, ganz besonders bevorzugt 0,3 bis 0,6 Gew.-%, jeweils bezogen auf Monomer a). Mit steigendem Vernetzergehalt sinkt die Zentrifugenretentionskapazität (CRC) und die Absorption unter einem Druck von 21,0 g/cm² durchläuft ein Maximum.

Als Initiatoren c) können sämtliche unter den Polymerisationsbedingungen Radikale erzeugende Verbindungen eingesetzt werden, beispielsweise thermische Initiatoren, Redox-Initiatoren, Photoinitiatoren. Geeignete Redox-Initiatoren sind Natriumperoxodisulfat/Ascorbinsäure, Wasserstoffperoxid/Ascorbinsäure, Natriumperoxodisulfat/Natriumbisulfit und Wasserstoffperoxid/Natriumbisulfit. Vorzugsweise werden Mischungen aus thermischen Initiatoren und Redox-Initiatoren eingesetzt, wie Natriumperoxodisulfat/Wasserstoffperoxid/Ascorbinsäure. Als reduzierende Komponente wird aber vorzugsweise ein Gemisch aus dem Natriumsalz der 2-Hydroxy-2-sulfinatoessigsäure, dem Dinatriumsalz der 2-Hydroxy-2-sulfonatoessigsäure und Natriumbisulfit eingesetzt. Derartige Gemische sind als Brüggolite® FF6 und Brüggolite® FF7 (Brüggemann Chemicals; Heilbronn; DE) erhältlich.

Mit den ethylenisch ungesättigten, säuregruppentragenden Monomeren a) copolymerisierbare ethylenisch ungesättigte Monomere d) sind beispielsweise Acrylamid, Methacrylamid, Hydroxyethylacrylat, Hydroxyethylmethacrylat, Dimethylaminoethylmethacrylat, Dimethylaminoethylacrylat, Dimethylaminopropylacrylat, Diethylaminopropylacrylat, Dimethylaminoethylmethacrylat, Diethylaminoethylmethacrylat.

Als wasserlösliche Polymere e) können Polyvinylalkohol, Polyvinylpyrrolidon, Stärke, Stärkederivate, modifizierte Cellulose, wie Methylcellulose oder Hydroxyethylcellulose, Gelatine, Polyglykole oder Polyacrylsäuren, vorzugsweise Stärke, Stärkederivate und modifizierte Cellulose, eingesetzt werden.

Üblicherweise wird eine wässrige Monomerlösung verwendet. Der Wassergehalt der Monomerlösung beträgt vorzugsweise von 40 bis 75 Gew.-%, besonders bevorzugt von 45 bis 70 Gew.-%, ganz besonders bevorzugt von 50 bis 65 Gew.-%. Es ist auch möglich Monomersuspensionen, d.h. Monomerlösungen mit überschüssigem Monomer a), beispielsweise Natriumacrylat, einzusetzen. Mit steigendem Wassergehalt steigt der Energieaufwand bei der anschließenden Trocknung und mit sinkendem Wassergehalt kann die Polymerisationswärme nur noch ungenügend abgeführt werden.

Die bevorzugten Polymerisationsinhibitoren benötigen für eine optimale Wirkung gelösten Sauerstoff. Daher kann die Monomerlösung vor der Polymerisation durch Inertisierung, d.h. Durchströmen mit einem inerten Gas, vorzugsweise Stickstoff oder Kohlendioxid, von gelöstem Sauerstoff befreit werden. Vorzugsweise wird der Sauerstoffgehalt der Monomerlösung vor der Polymerisation auf weniger als 1 Gew.-ppm, besonders bevorzugt auf weniger als 0,5 Gew.-ppm, ganz besonders bevorzugt auf weniger als 0,1 Gew.-ppm, gesenkt.

Geeignete Reaktoren sind beispielsweise Knetreaktoren oder Bandreaktoren. Im Kneter wird das bei der Polymerisation einer wässrigen Monomerlösung oder -suspension entstehende Polymergel durch beispielsweise gegenläufige Rührwellen kontinuierlich zerkleinert, wie in WO 2001/038402 A1 beschrieben. Die Polymerisation auf dem Band wird beispielsweise in DE 38 25 366 A1 und US 6,241,928 beschrieben. Bei der Polymerisation in einem Bandreaktor entsteht ein Polymergel, das in einem weiteren Verfahrensschritt zerkleinert werden muss, beispielsweise in einem Extruder oder Kneter.

Es ist aber auch möglich eine wässrige Monomerlösung zu vertropfen und die erzeugten Tropfen in einem erwärmten Trägergasstrom zu polymerisieren. Hierbei können die Verfahrensschritte Polymerisation und Trocknung zusammengefasst werden, wie in WO 2008/040715 A2 und WO 2008/052971 A1 beschrieben.

Die Säuregruppen der erhaltenen Polymergele sind üblicherweise teilweise neutralisiert. Die Neutralisation wird vorzugsweise auf der Stufe der Monomeren durchgeführt. Dies geschieht üblicherweise durch Einmischung des Neutralisationsmittels als wässrige Lösung oder bevorzugt auch als Feststoff. Der Neutralisationsgrad beträgt vorzugsweise von 25 bis 85 mol-%, für "saure" Polymergele besonders bevorzugt von 30 bis 60 mol-%, ganz besonders bevorzugt von 35 bis 55 mol-%, für "neutrale" Polymergele besonders bevorzugt von 65 bis 80 mol-%, ganz besonders bevorzugt von 70 bis 75 mol-%, wobei die üblichen Neutralisationsmittel verwendet werden können, vorzugsweise Alkalimetallhydroxide, Alkalimetalloxide, Alkalimetallkarbonate oder Alkalimetallhydrogenkarbonate sowie deren Mischungen. Statt Alkalimetallsalzen können auch Ammoniumsalze wie das Salz des Triethanolamins verwendet werden. Natrium und Kalium sind als Alkalimetalle besonders bevorzugt, ganz besonders bevorzugt sind jedoch Natriumhydroxid, Natriumkarbonat oder Natriumhydrogenkarbonat sowie deren Mischungen.

Es ist aber auch möglich die Neutralisation nach der Polymerisation auf der Stufe des bei der Polymerisation entstehenden Polymergels durchzuführen. Weiterhin ist es möglich bis zu 40 mol-%, vorzugsweise 10 bis 30 mol-%, besonders bevorzugt 15 bis 25 mol-%, der Säuregruppen vor der Polymerisation zu neutralisieren indem ein Teil des Neutralisationsmittels bereits der Monomerlösung zugesetzt und der gewünschte Endneutralisationsgrad erst nach der Polymerisation auf der Stufe des Polymergels eingestellt wird. Wird das Polymergel zumindest teilweise nach der Polymerisation neutralisiert, so wird das Polymergel vorzugsweise mechanisch zerkleinert, beispielsweise mittels eines Extruders, wobei das Neutralisationsmittel aufgesprüht, übergestreut oder aufgegossen und dann sorgfältig untergemischt werden kann. Dazu kann die erhaltene Gelmasse noch mehrmals zur Homogenisierung extrudiert werden.

Das Polymergel wird dann vorzugsweise mit einem Bandtrockner getrocknet bis der Restfeuchtegehalt vorzugsweise 0,5 bis 15 Gew.-%, besonders bevorzugt 1 bis 10 Gew.-%, ganz besonders bevorzugt 2 bis 8 Gew.-%, beträgt, wobei der Restfeuchtegehalt gemäß der von der EDANA (European Disposables and Nonwovens Association) empfohlenen Testmethode Nr. WSP 230.2-05 "Moisture Content" bestimmt wird. Bei einer zu hohen Restfeuchte weist das getrocknete Polymergel eine zu niedrige Glasübergangstemperatur T_{g} auf und ist nur schwierig weiter zu verarbeiten. Bei einer zu niedrigen Restfeuchte ist das getrocknete Polymergel zu spröde und in den anschließenden Zerkleinerungsschritten fallen unerwünscht große Mengen an Polymerpartikeln mit zu niedriger Partikelgröße ("fines") an. Der Feststoffgehalt des Gels beträgt vor der Trocknung vorzugsweise von 25 bis 90 Gew.-%, besonders bevorzugt von 35 bis 70 Gew.-%, ganz besonders bevorzugt von 40 bis 60 Gew.-%. Wahlweise kann zur Trocknung aber auch ein Wirbelbetttrockner oder ein Schaufeltrockner verwendet werden.

Das getrocknete Polymergel wird hiernach gemahlen und klassiert, wobei zur Mahlung üblicherweise ein- oder mehrstufige Walzenstühle, bevorzugt zwei- oder dreistufige Walzenstühle, Stiftmühlen, Hammermühlen oder Schwingmühlen, eingesetzt werden können.

Die mittlere Partikelgröße der als Produktfraktion abgetrennten Polymerpartikel beträgt vorzugsweise mindestens 200 µm, besonders bevorzugt von 250 bis 600 µm, ganz besonders von 300 bis 500 µm. Die mittlere Partikelgröße der Produktfraktion kann mittels der von der EDANA (European Disposables and Nonwovens Association) empfohlenen Testmethode Nr. WSP 220.2-05 "Partikel Size Distribution" ermittelt werden, wobei die Massenanteile der Siebfraktionen kumuliert aufgetragen werden und die mittlere Partikelgröße graphisch bestimmt wird. Die mittlere Partikelgröße ist hierbei der Wert der Maschenweite, der sich für kumulierte 50 Gew.-% ergibt.

Der Anteil an Partikeln mit einer Partikelgröße von mindestens 150 µm beträgt vorzugsweise mindestens 90 Gew.-%, besonders bevorzugt mindesten 95 Gew.-%, ganz besonders bevorzugt mindestens 98 Gew.-%.

Polymerpartikel mit zu niedriger Partikelgröße senken die Permeabilität (SFC). Daher sollte der Anteil zu kleiner Polymerpartikel ("fines") niedrig sein.

Zu kleine Polymerpartikel werden daher üblicherweise abgetrennt und in das Verfahren rückgeführt. Die geschieht vorzugsweise vor, während oder unmittelbar nach der Polymerisation, d.h. vor der Trocknung des Polymergels. Die zu kleinen Polymerpartikel können vor oder während der Rückführung mit Wasser und/oder wässrigem Tensid angefeuchtet werden.

Es ist auch möglich in späteren Verfahrensschritten zu kleine Polymerpartikel abzutrennen, beispielsweise nach der Oberflächennachvernetzung oder einem anderen Beschichtungsschritt. In diesem Fall sind die rückgeführten zu kleinen Polymerpartikel oberflächennachvernetzt bzw. anderweitig beschichtet, beispielsweise mit pyrogener Kieselsäure.

Wird zur Polymerisation ein Knetreaktor verwendet, so werden die zu kleinen Polymerpartikel vorzugsweise während des letzten Drittels der Polymerisation zugesetzt. Werden die zu kleinen Polymerpartikel sehr früh zugesetzt, beispielsweise bereits zur Monomerlösung, so wird dadurch die Zentrifugenretentionskapazität (CRC) der erhaltenen Superabsorberpartikel gesenkt. Dies kann aber beispielsweise durch Anpassung der Einsatzmenge an Vernetzer b) kompensiert werden.

Werden die zu kleinen Polymerpartikel sehr spät zugesetzt, beispielsweise erst in einem dem Polymerisationsreaktor nachgeschalteten Apparat, beispielsweise einem Extruder, so lassen sich die zu kleinen Polymerpartikel nur noch schwer in das erhaltene Polymergel einarbeiten. Unzureichend eingearbeitete zu kleine Polymerpartikel lösen sich aber während der Mahlung wieder von dem getrockneten Polymergel, werden beim Klassieren daher erneut abgetrennt und erhöhen die Menge rückzuführender zu kleiner Polymerpartikel.

Der Anteil an Partikeln mit einer Partikelgröße von höchstens 850 µm, beträgt vorzugsweise mindestens 90 Gew.-%, besonders bevorzugt mindesten 95 Gew.-%, ganz besonders bevorzugt mindestens 98 Gew.-%.

Der Anteil an Partikeln mit einer Partikelgröße von höchstens 600 µm, beträgt vorzugsweise mindestens 90 Gew.-%, besonders bevorzugt mindesten 95 Gew.-%, ganz besonders bevorzugt mindestens 98 Gew.-%.

Polymerpartikel mit zu großer Partikelgröße senken die Anquellgeschwindigkeit. Daher sollte der Anteil zu großer Polymerpartikel ebenfalls niedrig sein.

Zu große Polymerpartikel werden daher üblicherweise abgetrennt und in die Mahlung des getrockneten Polymergels rückgeführt.

Die Polymerpartikel können zur weiteren Verbesserung der Eigenschaften oberflächennachvernetzt werden. Geeignete Oberflächennachvernetzer sind Verbindungen, die Gruppen enthalten, die mit mindestens zwei Carboxylatgruppen der Polymerpartikel kovalente Bindungen bilden können. Geeignete Verbindungen sind beispielsweise polyfunktionelle Amine, polyfunktionelle Amidoamine, polyfunktionelle Epoxide, wie in EP 0 083 022 A2, EP 0 543 303 A1 und EP 0 937 736 A2 beschrieben, di- oder polyfunktionelle Alkohole, wie in DE 33 14 019 A1, DE 35 23 617 A1 und EP 0 450 922 A2 beschrieben, oder β-Hydroxyalkylamide, wie in DE 102 04 938 A1 und US 6,239,230 beschrieben.

Des weiteren sind in DE 40 20 780 C1 zyklische Karbonate, in DE 198 07 502 A1 2-Oxazolidon und dessen Derivate, wie 2-Hydroxyethyl-2-oxazolidon, in DE 198 07 992 C₁ Bis- und Poly-2-oxazolidinone, in DE 198 54 573 A1 2-Oxotetrahydro-1,3-oxazin und dessen Derivate, in DE 198 54 574 A1 N-Acyl-2-Oxazolidone, in DE 102 04 937 A1 zyklische Harnstoffe, in DE 103 34 584 A1 bizyklische Amidacetale, in EP 1 199 327 A2 Oxetane und zyklische Harnstoffe und in WO 2003/031482 A1 Morpholin-2,3-dion und dessen Derivate als geeignete Oberflächennachvernetzer beschrieben.

Bevorzugte Oberflächennachvernetzer sind Ethylenkarbonat, Ethylenglykoldiglycidylether, Umsetzungsprodukte von Polyamiden mit Epichlorhydrin und Gemische aus Propylenglykol und 1,4-Butandiol.

Ganz besonders bevorzugte Oberflächennachvernetzer sind 2-Hydroxyethyloxazolidin-2-on, Oxazolidin-2-on und 1,3-Propandiol.

Weiterhin können auch Oberflächennachvernetzer eingesetzt werden, die zusätzliche polymerisierbare ethylenisch ungesättigte Gruppen enthalten, wie in DE 37 13 601 A1 beschrieben

Die Menge an Oberflächennachvernetzer beträgt vorzugsweise 0,001 bis 2 Gew.-%, besonders bevorzugt 0,02 bis 1 Gew.-%, ganz besonders bevorzugt 0,05 bis 0,2 Gew.-%, jeweils bezogen auf die Polymerpartikel.

In einer bevorzugten Ausführungsform der vorliegenden Erfindung werden vor, während oder nach der Oberflächennachvernetzung zusätzlich zu den Oberflächennachvernetzern polyvalente Kationen auf die Partikeloberfläche aufgebracht.

Die im erfindungsgemäßen Verfahren einsetzbaren polyvalenten Kationen sind beispielsweise zweiwertige Kationen, wie die Kationen von Zink, Magnesium, Kalzium und Strontium, dreiwertige Kationen, wie die Kationen von Aluminium, vierwertige Kationen, wie die Kationen von Titan und Zirkonium. Als Gegenion sind beispielsweise Chlorid, Bromid, Sulfat, Hydrogensulfat, Carbonat, Hydrogencarbonat, Nitrat, Phosphat, Hydrogenphosphat, Dihydrogenphosphat und Carboxylat, wie Acetat und Lactat, möglich. Aluminiumsulfat und Aluminiumlaktat sind bevorzugt. Außer Metallsalzen können auch Polyamine als polyvalente Kationen eingesetzt werden.

Die Einsatzmenge an polyvalentem Kation beträgt beispielsweise 0,001 bis 1,5 Gew.-%, vorzugsweise 0,005 bis 1 Gew.-%, besonders bevorzugt 0,02 bis 0,8 Gew.-%. jeweils bezogen auf die Polymerpartikel.

Die Oberflächennachvernetzung wird üblicherweise so durchgeführt, dass eine Lösung des Oberflächennachvernetzers auf die getrockneten Polymerpartikel aufgesprüht wird. Im Anschluss an das Aufsprühen werden die mit Oberflächennachvernetzer beschichteten Polymerpartikel thermisch getrocknet, wobei die Oberflächennachvernetzungsreaktion sowohl vor als auch während der Trocknung stattfinden kann.
Das Aufsprühen einer Lösung des Oberflächennachvernetzers wird vorzugsweise in Mischern mit bewegten Mischwerkzeugen, wie Schneckenmischer, Scheibenmischer und Schaufelmischer, durchgeführt werden. Besonders bevorzugt sind Horizontalmischer, wie Schaufelmischer, ganz besonders bevorzugt sind Vertikalmischer. Die Unterscheidung in Horizontalmischer und Vertikalmischer erfolgt über die Lagerung der Mischwelle, d.h. Horizontalmischer haben eine horizontal gelagerte Mischwelle und Vertikalmischer haben eine vertikal gelagerte Mischwelle. Geeignete Mischer sind beispielsweise Horizontale Pflugschar® Mischer (Gebr. Lödige Maschinenbau GmbH; Paderborn; DE), Vrieco-Nauta Continuous Mixer (Hosokawa Micron BV; Doetinchem; NL), Processall Mixmill Mixer (Processall Incorporated; Cincinnati; US) und Schugi Flexomix® (Hosokawa Micron BV; Doetinchem; NL). Es ist aber auch möglich die O-berflächennachvernetzerlösung in einem Wirbelbett aufzusprühen.

Die Oberflächennachvernetzer werden typischerweise als wässrige Lösung eingesetzt. Über den Gehalt an nichtwässrigem Lösungsmittel bzw. Gesamtlösungsmittelmenge kann die Eindringtiefe des Oberflächennachvernetzers in die Polymerpartikel eingestellt werden.

Wird ausschließlich Wasser als Lösungsmittel verwendet, so wird vorteilhaft ein Tensid zugesetzt. Dadurch wird das Benetzungsverhalten verbessert und die Verklumpungsneigung vermindert. Vorzugsweise werden aber Lösungsmittelgemische eingesetzt, beispielsweise Isopropanol/Wasser, 1,3-Propandiol/Wasser und Propylenglykol/Wasser, wobei das Mischungsmassenverhältnis vorzugsweise von 20:80 bis 40:60 beträgt.
Die thermische Trocknung wird vorzugsweise in Kontakttrocknern, besonders bevorzugt Schaufeltrocknern, ganz besonders bevorzugt Scheibentrocknern, durchgeführt. Geeignete Trockner sind beispielsweise Hosokawa Bepex® Horizontal Paddle Dryer (Hosokawa Micron GmbH; Leingarten; DE), Hosokawa Bepex® Disc Dryer (Hosokawa Micron GmbH; Leingarten; DE) und Nara Paddle Dryer (NARA Machinery Europe; Frechen; DE). Überdies können auch Wirbelschichttrockner eingesetzt werden.

Die Trocknung kann im Mischer selbst erfolgen, durch Beheizung des Mantels oder Einblasen von Warmluft. Ebenso geeignet ist ein nachgeschalteter Trockner, wie beispielsweise ein Hordentrockner, ein Drehrohrofen oder eine beheizbare Schnecke. Besonders vorteilhaft wird in einem Wirbelschichttrockner gemischt und getrocknet.

Bevorzugte Trocknungstemperaturen liegen im Bereich 100 bis 250°C, bevorzugt 120 bis 220°C, besonders bevorzugt 130 bis 210°C, ganz besonders bevorzugt 150 bis 200°C. Die bevorzugte Verweilzeit bei dieser Temperatur im Reaktionsmischer oder Trockner beträgt vorzugsweise mindestens 10 Minuten, besonders bevorzugt mindestens 20 Minuten, ganz besonders bevorzugt mindestens 30 Minuten, und üblicherweise höchstens 60 Minuten.

Anschließend können die oberflächennachvernetzten Polymerpartikel erneut klassiert werden, wobei zu kleine und/oder zu große Polymerpartikel abgetrennt und in das Verfahren rückgeführt werden.

Die oberflächennachvernetzten Polymerpartikel können zur weiteren Verbesserung der Eigenschaften beschichtet oder nachbefeuchtet werden.

Die Nachbefeuchtung wird vorzugsweise bei 30 bis 80°C, besonders bevorzugt bei 35 bis 70°C, ganz besonders bevorzugt bei 40 bis 60°C, durchgeführt. Bei zu niedrigen Temperaturen neigen die Superabsorberpartikel zum Verklumpen und bei höheren Temperaturen verdampft bereits merklich Wasser. Die zur Nachbefeuchtung eingesetzte Wassermenge beträgt vorzugsweise von 1 bis 10 Gew.-%, besonders bevorzugt von 2 bis 8 Gew.-%, ganz besonders bevorzugt von 3 bis 5 Gew.-%. Durch die Nachbefeuchtung wird die mechanische Stabilität der Polymerpartikel erhöht und deren Neigung zur statischen Aufladung vermindert.

Geeignete Beschichtungen zur Verbesserung der Anquellgeschwindigkeit sowie der Permeabilität (SFC) sind beispielsweise anorganische inerte Substanzen, wie wasserunlösliche Metallsalze, organische Polymere, kationische Polymere sowie zwei- oder mehrwertige Metallkationen. Geeignete Beschichtungen zur Staubbindung sind beispielsweise Polyole. Geeignete Beschichtungen gegen die unerwünschte Verbackungsneigung der Polymerpartikel sind beispielsweise pyrogene Kieselsäure, wie Aerosil® 200, und Tenside, wie Span® 20.

Die Superabsorberpartikel weisen einen Feuchtegehalt von vorzugsweise 1 bis 15 Gew.-%, besonders bevorzugt 2 bis 10 Gew.-%, ganz besonders bevorzugt 3 bis 5 Gew.-%, auf, wobei der Feuchtegehalt gemäß der von der EDANA (European Disposables and Nonwovens Association) empfohlenen Testmethode Nr. WSP 230.2-05 "Moisture Content" bestimmt wird.

Die Superabsorberpartikel weisen eine Zentrifugenretentionskapazität (CRC) von typischerweise mindestens 15 g/g, vorzugsweise mindestens 20 g/g, bevorzugt mindestens 22 g/g, besonders bevorzugt mindestens 24 g/g, ganz besonders bevorzugt mindestens 26 g/g, auf. Die Zentrifugenretentionskapazität (CRC) der Superabsorberpartikel beträgt üblicherweise weniger als 60 g/g. Die Zentrifugenretentionskapazität (CRC) wird gemäß der von der EDANA (European Disposables and Nonwovens Association) empfohlenen Testmethode Nr. WSP 241.2-05 "Centrifuge Retention Capacity" bestimmt.

Die Superabsorberpartikel weisen eine Absorption unter einem Druck von 49,2 g/cm² von typischerweise mindestens 15 g/g, vorzugsweise mindestens 20 g/g, bevorzugt mindestens 22 g/g, besonders bevorzugt mindestens 24 g/g, ganz besonders bevorzugt mindestens 26 g/g, auf. Die Absorption unter einem Druck von 49,2 g/cm² der Superabsorberpartikel beträgt üblicherweise weniger als 35 g/g. Die Absorption unter einem Druck von 49,2 g/cm² wird analog der von der EDANA (European Disposables and Nonwovens Association) empfohlenen Testmethode Nr. WSP 242.2-05 "Absorption under Pressure" bestimmt, wobei statt eines Drucks von 21,0 g/cm² ein Druck von 49,2 g/cm² eingestellt wird.

Ein weiterer Gegenstand der vorliegenden Erfindung sind Verfahren zur Herstellung der erfindungsgemäßen Zusammensetzungen, wobei
i) das mindestens eine Metallperoxid mit Superabsorberpartikeln zusammen gemischt wird und/oder
ii) das mindestens eine Metallperoxid mit Superabsorberpartikeln zusammen gemahlen wird und/oder
iii) das mindestens eine Metallperoxid auf Superabsorberpartikel aufgesprüht wird und
iv) optional die gemäß i), ii) und/oder iii) erhaltene Zusammensetzung mit Superabsorberpartikeln zusammen gemischt wird.

Die Variante i) ist bevorzugt.

Die Art des Mischens unterliegt keiner Beschränkung und kann bereits bei der Herstellung der Superabsorberpartikel, beispielsweise beim Kühlen nach der Oberflächennachvernetzung oder dem anschließenden Klassieren, oder in einem speziellen Mischer erfolgen. Geeignete Mischer wurden bereits oben bei der Oberflächennachvernetzung der Superabsorberpartikel beschrieben.

Die Art des Mahlens unterliegt ebenfalls keiner Beschränkung. Geeignete Apparate wurden bereits oben bei der Zerkleinerung der Superabsorberpartikel beschrieben.

Die Art des Aufsprühens unterliegt keiner Beschränkung.

Bei der Herstellung von Pulvermischungen aus Superabsorberpartikeln und mindestens einem Metallperoxid werden vorteilhaft Entstaubungsmittel eingesetzt. Geeignete Entstaubungsmittel sind Polyglyzerine, Polyethylenglykole, Polyproplenglykole, statistische oder Block-Copolymere von Ethylenoxid und Propylenoxid. Weitere zu diesem Zweck geeignete Entstaubungsmittel sind die Polyethoxylate oder Poly-propoxylate von Polyhydroxyverbindungen, wie Glyzerin, Sorbitol, Trimethylolpropan, Trimethylolethan und Pentaerythrit. Beispiele hierfür sind n-fach ethoxyliertes Trimethylolpropan oder Glyzerin, wobei n eine ganze Zahl zwischen 1 und 100 darstellt. Weitere Beispiele sind Block-Copolymere, wie insgesamt n-fach ethoxyliertes und dann m-fach propoxyliertes Trimethylolpropan oder Glyzerin, wobei n eine ganze Zahl zwischen 1 und 40 und m eine ganze Zahl zwischen 1 und 40 darstellt. Die Reihenfolge der Blöcke kann auch umgekehrt sein. Die Entstaubungsmittel können auch mit Wasser verdünnt werden.

Bei Pulvermischungen ist die Dichte der zu mischenden Komponenten wichtig. Es gibt dann keine Entmischung, wenn die Dichten von wasserabsorbierenden Polymerpartikeln und Metallperoxid ähnlich sind oder die Partikelgröße des Metallperoxids viel kleiner ist als die der Superabsorberpartikel.

Ein weiterer Gegenstand der vorliegenden Erfindung sind Hygieneartikel, enthaltend mindestens eine erfindungsgemäße Zusammensetzung, insbesondere Hygieneartikel zur Damenhygiene, Hygieneartikel für leichte und schwere Inkontinenz oder Kleintierstreu.

Die Hygieneartikel enthalten üblicherweise eine wasserundurchlässige Rückseite, eine wasserdurchlässige Oberseite und dazwischen einen absorbierenden Kern aus dem erfindungsgemäßen Superabsorberpartikeln und Fasern, vorzugsweise Cellulose. Der Anteil der erfindungsgemäßen Superabsorberpartikel im absorbierenden Kern beträgt vorzugsweise 20 bis 100 Gew.-%, bevorzugt 50 bis 100 Gew.-%.

Die Superabsorberpartikel werden mittels der nachfolgend beschriebenen Testmethoden geprüft.

### Methoden:

Die Messungen sollten, wenn nicht anders angegeben, bei einer Umgebungstemperatur von 23 ± 2 °C und einer relativen Luftfeuchte von 50 ± 10 % durchgeführt werden. Die Superabsorberpartikel werden vor der Messung gut durchmischt.

### Zentrifugenretentionskapazität (Centrifuge Retention Capacity)

Die Zentrifugenretentionskapazität (CRC) wird gemäß der von der EDANA empfohlenen Testmethode Nr. WSP 241.2-05 "Centrifuge Retention Capacity" bestimmt.

### Bakterieninduzierte Ammoniakfreisetzung

DSM1 Medium (Deutsche Sammlung von Mikroorganismen und Zellkulturen GmbH) wurde hergestellt aus 5,0 g/l Pepton aus Fleisch (Merck KGaA; Darmstadt; DE; Art. Nr. 1.07214) und 3,0 g/l Fleischextrakt (Merck KGaA; Darmstadt; DE; Art. Nr. 1103979) und auf pH = 7,0 eingestellt. 50 ml DSM1 Medium wurde mit Proteus mirabilis ATCC 14153 auf OD = 0,1 angeimpft und in einem 250 ml Erlenmeyerkolben mit Schikane für 15 Stunden bei 37°C und 220 Upm inkubiert. Die so hergestellten Kulturen hatten eine Zelldichte von ungefähr 10⁹ KBE/ml (OD = 2,0 - 2,5).

Der synthetische Urin wurde hergestellt aus 25 g/l Harnstoff (sterilfiltriert), 9,0 g/l Natriumchlorid, 1 g/l Pepton aus Fleisch und 1 g/l Fleischextrakt. Der synthetische Urin wurde vor Zugabe einer sterilfiltrierten konzentrierten Harnstofflösung autoklaviert.

125 ml Polypropylen-Histologiebecher wurden autoklaviert und die zur Aufnahme von 50 ml synthetischem Urin notwendige Menge Superabsorberpartikel vorgelegt (berechnet aus der Zentrifugenretentionskapazität). Dann wurden 50 ml synthetischer Urin mit 50 µL Bakterienstammlösung entsprechend einer Gesamtkonzentration von ca. 10⁶ KBE/ml beimpft, mit den wasserabsorbierenden Polymerpartikeln vermischt und sofort der mit einem Diffusionsteströhrchen (Drägerwerk AG & Co. KGaA; Lübeck; DE; Dräger-Röhrchen® Ammoniak 20/a-D; Sach-Nr. 8101301) versehene Deckel aufgeschraubt. Die Ammoniakentwicklung wurde bei 37°C über 48 Stunden beobachtet.

### Messung der Lagerstabilität

Ca. 10 g der jeweiligen Superabsorberpartikel werden in ein Schnappdeckelglas (ca. 50 ml) gegeben und ohne Deckel bei 80°C im Umlufttrockenschrank für 14 Stunden gelagert. Anschließend werden die Gläser sofort verschlossen und auf Umgebungstemperatur abgekühlt.

Der Peroxidgehalt wird nach der in "Deutsche Einheitsmethoden zur Untersuchung von Fetten, Fettprodukten, Tensiden und verwandten Stoffen", bearbeitet und herausgegeben von der Deutschen Gesellschaft für Fettwissenschaft e.V., 2. Auflage einschließlich 9. Erg.-Lfg. Band 2 (Wissenschaftliche Verlagsgesellschaft mbH, Stuttgart, 2004)

Die Ergebnisse werden relativ zu einer geschlossenen, bei Umgebungstemperatur gelagerten Probe angegeben.

### Beispiele

Die Beispiele wurden mit Hysorb® B7055 bzw. Hysorb® B7065 (BASF SE; Ludwigshafen; DE), handelsüblichen oberflächennachvernetzten Superabsorberpartikeln auf Natriumacrylatbasis mit einem Neutralisationsgrad von 70 bis 75 mol-%, durchgeführt.

Derartige oberflächennachvernetzte Superabsorberpartikel sind z.B. von BASF Aktiengesellschaft (Handelsname HySorb®), von Stockhausen GmbH (Handelsname Favor®) und von Nippon Shokubai Co., Ltd. (Handelsname Aqualic®) kommerziell erhältlich.

### Beispiel 1

20g Superabsorberpartikel (HySorb® B7065; BASF SE; DE) wurden mit 0,2g Zinkperoxid (55 gew.-%ig; VWR International LLC; Buffalo Grove; US) in eine 50ml Glasflasche eingewogen. Anschließend wurde die Mischung in den großen Porzellanmörser (16cm Innendurchmesser) überführt und dort für ca. 5 Minuten verrieben. Zusätzlich wurden die Proben nochmals 20 Minuten bei 46Upm im Taumelmischer homogenisiert.

Die erhaltene Zusammensetzung wurde analysiert. Die Ergebnisse sind in Tabelle 1 zusammengefasst:

**Tab. 1: Zusatz von Zinkperoxid**

| Beispiel | CRC [g/g] | Zeit bis zum Erreichen von 1.500 ppm·h Ammoniak oder Wert nach 48h |
|---|---|---|
| Hysorb® B7065 | 29,9 | 8,25h |
| 1 | 29,5 | kein Ammoniak mehr nachweisbar |

### Beispiel 2 (nicht erfindungsgemäß)

300g Superabsorberpartikel (HySorb® B7055; BASF SE; DE) wurden in eine handelsübliche Küchenmaschine (Bosch Profi Mixx 47, E-Nr. MUM4700/05, Stufe 3, Rührbesen) eingefüllt. 6,12g einer Lösung (163,3g 30%iges Wasserstoffperoxid mit dest. Wasser auf 1000g aufgefüllt) wurde mit einem Sprühzerstäuber (800 l/h Stickstoff) aufgesprüht. Die Mischung wurde weitere 10 Minuten bei Stufe 1 gerührt.

Die erhaltene Zusammensetzung wurde analysiert. Die Ergebnisse sind in Tabelle 2 zusammengefasst:

### Beispiel 3 (nicht erfindungsgemäß)

300g Superabsorberpartikel (HySorb® B7055; BASF SE; DE) wurden in eine handelsübliche Küchenmaschine (Bosch Profi Mixx 47, E-Nr. MUM4700/05, Stufe 3, Rührbesen) eingefüllt. 6,12g einer Lösung (156,59g Wasserstoffperoxid-Harnstoffaddukt mit dest. Wasser auf 1000g aufgefüllt) wurde mit einem Sprühzerstäuber (800 l/h Stickstoff) aufgesprüht. Die Mischung wurde weitere 10 Minuten bei Stufe 3 gerührt.

Die erhaltene Zusammensetzung wurde analysiert. Die Ergebnisse sind in Tabelle 2 zusammengefasst:

### Beispiel 4

20g Superabsorberpartikel (HySorb® B7055; BASF SE; DE) wurden mit 0,383g Zinkperoxid (71,6 gew.-%ig; ShanPar Industries Pvt. Ltd.; IN) in eine 50ml Glasflasche eingewogen. Anschließend wurde diese Mischung in einen Porzellanmörser (8cm Innendurchmesser) überführt und dort verrieben. Die verrieben Mischung wurde in eine 500ml Vierkantplastikflasche überführt und weitere 250g Superabsorberpartikel (HySorb® B7055; BASF SE; DE) zugeben. Zusätzlich wurde die Probe nochmals 20 Minuten bei 46Upm im Taumelmischer homogenisiert. Diese Mischung wurde in eine handelsübliche Küchenmaschine (Bosch Profi Mixx 47, E-Nr. MUM4700/05, Stufe 3, Rührbesen) eingefüllt und mit 5,96g einer Mischung aus Polyethylenglykol 400 und Wasser (50:50 m/m) mit einem Sprühzerstäuber (800 l/h Stickstoff) besprüht. Die Mischung wurde weitere 10 Minuten bei Stufe 3 gerührt.

Die erhaltene Zusammensetzung wurde analysiert. Die Ergebnisse sind in Tabelle 2 zusammengefasst:

### Beispiel 5

20g Superabsorberpartikel (HySorb® B7055; BASF SE; DE) mit 1,044g Magnesiumperoxid (26 gew.-%ig; Sigma Aldrich) in eine 50ml Glasflasche eingewogen. Anschließend wurde diese Mischung in einen Porzellanmörser (8cm Innendurchmesser) überführt und dort verrieben. Die verrieben Mischung wurde in eine 500ml Vierkantplastikflasche überführt und weiter 250g Superabsorberpartikel (HySorb® B7055; BASF SE; DE) zugeben. Zusätzlich wurde die Probe nochmals 20 Minuten bei 46Upm im Taumelmischer homogenisiert. Diese Mischung wurde in eine handelsübliche Küchenmaschine (Bosch Profi Mixx 47, E-Nr. MUM4700/05, Stufe 3, Rührbesen) eingefüllt und mit 5,77g einer Mischung aus Polyethylenglykol 400 und
Wasser (50:50 m/m) mit einem Sprühzerstäuber (800 l/h Stickstoff) besprüht. Die Mischung wurde weitere 10 Minuten bei Stufe 3 gerührt.
Die erhaltene Zusammensetzung wurde analysiert. Die Ergebnisse sind in Tabelle 2 zusammengefasst:

### Beispiel 6

20g Superabsorberpartikel (HySorb® B7055; BASF SE; DE) wurden mit 0,360g Kalziumperoxid (75 gew.-%ig; Sigma Aldrich) in eine 50ml Glasflasche eingewogen. Anschließend wurde diese Mischung in einen Porzellanmörser (8cm Innendurchmesser) überführt und dort verrieben. Die verrieben Mischung wurde in eine 500ml Vierkantplastikflasche überführt und weitere 250g Superabsorberpartikel (HySorb® B7055; BASF SE; DE) zugeben. Zusätzlich wurde die Probe nochmals 20 Minuten bei 46Upm im Taumelmischer homogenisiert. Diese Mischung wurde in eine handelsübliche Küchenmaschine (Bosch Profi Mixx 47,
E-Nr. MUM4700/05, Stufe 3, Rührbesen) eingefüllt und mit 5,79g einer Mischung aus Polyethylenglykol 400 und Wasser (50:50 m/m) mit einem Sprühzerstäuber (800 l/h Stickstoff) besprüht. Die Mischung wurde weitere 10 Minuten bei Stufe 3 gerührt.
Die erhaltene Zusammensetzung wurde analysiert. Die Ergebnisse sind in Tabelle 2 zusammengefasst:

### Beispiel 7 (nicht erfindungsgemäß)

20g Superabsorberpartikel (HySorb® B7055; BASF SE; DE) wurden mit 0,300g Lithiumperoxid (90 gew.-%ig; Sigma Aldrich) in eine 50ml Glasflasche eingewogen. Anschließend wurde diese Mischung in einen Porzellanmörser (8cm Innendurchmesser) überführt und dort verrieben. Die verriebene Mischung wurde in eine 500ml Vierkantplastikflasche überführt und weitere 250g Superabsorberpartikel (HySorb® B7055; BASF SE; DE) zugeben. Zusätzlich wurden die Probe nochmals 20 Minuten bei 46Upm im Taumelmischer homogenisiert. Diese Mischung wurde in eine handelsübliche Küchenmaschine (Bosch Profi Mixx 47,
E-Nr. MUM4700/05, Stufe 3, Rührbesen) eingefüllt und mit 5,62g einer Mischung aus Polyethylenglykol 400 und Wasser (50:50 m/m) mit einem Sprühzerstäuber (800 l/h Stickstoff) besprüht. Die Mischung wurde weitere 10 Minuten bei Stufe 3 gerührt.
Die erhaltene Zusammensetzung wurde analysiert. Die Ergebnisse sind in Tabelle 2 zusammengefasst:

**Tab. 2: Messung der Lagerstabilität**

| Beispiel | Metallperoxid | Peroxidgehaltes nach der Lagerung |
|---|---|---|
| 2 | Wasserstoffperoxid | 80% ± 5% |
| 3 | Wasserstoffperoxid/Harnstoff | 77% ± 5% |
| 4 | Zinkperoxid | 104% ± 5% |
| 5 | Magnesiumperoxid | 94% ± 5% |
| 6 | Kalziumperoxid | 93% ± 5% |
| 7 | Lithiumperoxid | 64% ± 5% |

## Patentansprüche

1. Geruchsinhibierende Zusammensetzung, enthaltend mindestens 90 Gew.-% Superabsorberpartikel und mindestens ein Metallperoxid, das Zinkperoxid, Magnesiumperoxid oder Kalziumperoxid ist.

2. Zusammensetzung gemäß Anspruch 1, **dadurch gekennzeichnet, dass** das Metallperoxid Zinkperoxid ist.

3. Zusammensetzung gemäß einem der Ansprüche 1 bis 2, **dadurch gekennzeichnet, dass** die Zusammensetzung von 0,001 bis 5 Gew.-% des Metallperoxids enthält.

4. Zusammensetzung gemäß einem der Ansprüche 1 bis 3, **dadurch gekennzeichnet, dass** die Zusammensetzung weniger als 8 Gew.-% Wasser enthält.

5. Zusammensetzung gemäß einem der Ansprüche 1 bis 4, **dadurch gekennzeichnet, dass** die Superabsorberpartikel zu mindestens 50 Gew.-% polymerisierte Acrylsäure und/oder deren Salze enthalten.

6. Zusammensetzung gemäß einem der Ansprüche 1 bis 5, **dadurch gekennzeichnet, dass** die Superabsorberpartikel eine Zentrifugenretentionskapazität von mindestens 15 g/g aufweisen.

7. Verfahren zur Herstellung von in einem der Ansprüche 1 bis 6 definierter Zusammensetzungen, **dadurch gekennzeichnet, dass** man mindestens einen der folgenden Schritte durchführt, wobei
i) das mindestens eine Metallperoxid mit Superabsorberpartikeln zusammen gemischt wird und/oder
ii) das mindestens eine Metallperoxid mit Superabsorberpartikeln zusammen gemahlen wird und/oder
iii) das mindestens eine Metallperoxid auf Superabsorberpartikel aufgesprüht wird und
iv) optional die gemäß i), ii) und/oder iii) erhaltene Zusammensetzung mit weiterem Superabsorberpartikeln zusammen gemischt werden.

8. Hygieneartikel, enthaltend mindestens eine Zusammensetzung gemäß einem der Ansprüche 1 bis 6.

9. Hygieneartikel gemäß Anspruch 8, **dadurch gekennzeichnet, dass** der Hygieneartikel ein Artikel zur Damenhygiene, ein Artikel für leichte und schwere Inkontinenz oder Kleintierstreu ist.

## Claims

1. An odor-inhibiting composition comprising at least 90% by weight of superabsorbent particles and at least one metal peroxide which is zinc peroxide, magnesium peroxide or calcium peroxide.

2. The composition according to claim 1, wherein the metal peroxide is zinc peroxide.

3. The composition according to either of claims 1 and 2, wherein the composition comprises from 0.001 to 5% by weight of the metal peroxide.

4. The composition according to any of claims 1 to 3, wherein the composition comprises less than 8% by weight of water.

5. The composition according to any of claims 1 to 4, wherein the superabsorbent particles comprise at least 50% by weight of polymerized acrylic acid and/or the salts thereof.

6. The composition according to any of claims 1 to 5, wherein the superabsorbent particles have a centrifuge retention capacity of at least 15 g/g.

7. A process for producing compositions defined in any of claims 1 to 6, which comprises performing at least one of the following steps:
i) mixing the at least one metal peroxide together with superabsorbent particles and/or
ii) grinding the at least one metal peroxide together with superabsorbent particles and/or
iii) spraying the at least one metal peroxide onto superabsorbent particles and
iv) optionally mixing the composition obtained in i), ii) and/or iii) together with further superabsorbent particles.

8. A hygiene article comprising at least one composition according to any of claims 1 to 6.

9. The hygiene article according to claim 8, wherein the hygiene article is an article for feminine hygiene, an article for light and heavy incontinence, or small animal litter.

## Revendications

1. Composition inhibitrice d'odeur, contenant au moins 90 % en poids de particules superabsorbantes et au moins un peroxyde métallique, qui est le peroxyde de zinc, le peroxyde de magnésium ou le peroxyde de calcium.

2. Composition selon la revendication 1, **caractérisée en ce que** le peroxyde métallique est le peroxyde de zinc.

3. Composition selon l'une des revendications 1 à 2, **caractérisée en ce que** la composition contient de 0,001 à 5 % en poids du peroxyde métallique.

4. Composition selon l'une des revendications 1 à 3, **caractérisée en ce que** la composition contient moins de 8 % en poids d'eau.

5. Composition selon l'une des revendications 1 à 4, **caractérisée en ce que** les particules superabsorbantes contiennent de l'acide acrylique polymérisé à au moins 50 % en poids, et/ou des sels de ce dernier.

6. Composition selon l'une des revendications 1 à 5, **caractérisée en ce que** les particules superabsorbantes présentent une capacité de rétention à la centrifugeuse d'au moins 15 g/g.

7. Procédé de fabrication de compositions définies dans l'une des revendications 1 à 6, **caractérisé en ce qu'**on met en oeuvre au moins l'une des étapes suivantes, dans lesquelles
i) le ou les peroxydes métalliques sont mélangés à des particules superabsorbantes, et/ou
ii) le ou les peroxydes métalliques sont broyés avec les particules superabsorbantes, et/ou
iii) le ou les peroxydes métalliques sont pulvérisés sur les particules superabsorbantes, et
iv) en option, la composition obtenue selon i), ii) et/ou iii) est mélangée à d'autres particules superabsorbantes.

8. Article hygiénique, contenant au moins une composition selon l'une des revendications 1 à 6.

9. Article hygiénique selon la revendication 8, **caractérisé en ce que** l'article hygiénique est un article pour l'hygiène féminine, un article pour une incontinence légère ou grave, ou une litière pour petits animaux.
